# EUROPEAN PATENT APPLICATION

(11) **EP 2 905 052 A1**
(43) Date of publication of application: **12.08.2015**
(21) Application number: 15151778.6
(22) Date of filing: 20.01.2015
(51) Int. Cl.: A61N 5/06

(54) **HAIR GROWTH INHIBITION APPARATUS AND HAIR GROWTH INHIBITION METHOD**

(30) Priority: 07.02.2014 JP 2014022421
(71) Applicant: Panasonic Intellectual Property Management Co., Ltd., Osaka-shi, Osaka 540-6207 (JP)
(72) Inventor: Ajiki, Kaori, Osaka-shi, Osaka 540-6207 (JP)
(74) Representative: Grünecker Patent- und Rechtsanwälte PartG mbB

(57) **Abstract**

A hair growth inhibition apparatus performs a hair growth inhibition process on skin by using light. The hair growth inhibition apparatus includes a sheet member capable of being closely attached to the skin, a temperature sensor that is disposed on the sheet member and detects a temperature of the skin in a region around the temperature sensor, and a light emitting element that is disposed on the sheet member at a location corresponding to a location of the temperature sensor and emits the light toward the skin in the region around the temperature sensor in accordance with a detection result obtained by the temperature sensor.

## Description

### CROSS REFERENCES TO RELATED APPLICATIONS

This application claims priority to Japanese Patent Application No. 2014-022421 filed on February 7, 2014, the contents of which are hereby incorporated by reference.

### BACKGROUND

### 1. Technical Field

The present disclosure relates to a hair growth inhibition apparatus and a hair growth inhibition method.

### 2. Description of the Related Art

Japanese Unexamined Patent Application Publication (Translation of PCT Application) No. 2004-529705, for example, describes an apparatus that performs a hair growth inhibition process on skin by irradiating the skin with light (hereinafter referred to as a "hair growth inhibition apparatus").

The hair growth inhibition apparatus described in Japanese Unexamined Patent Application Publication (Translation of PCT Application) No. 2004-529705 (hereinafter referred to as the related art) includes a housing which has a handle and in which a radiation source and a detector are disposed. The radiation source outputs optical radiation pulses. The detector detects the temperature of the skin irradiated with light. According to the related art, pulse dose is controlled on the basis of the detected temperature of the skin.

For example, a user places an exit opening of the radiation pulses on an area to be subjected to the hair growth inhibition process. Thus, according to the related art, skin can be subjected to the hair growth inhibition process without an effect of temperature increase, except for hair growth inhibition.

However, according to the related art, a large burden is placed on the user.

### SUMMARY

One non-limiting and exemplary embodiment provides a hair growth inhibition apparatus capable of reducing the burden on the user in a hair growth inhibition process.

Additional benefits and advantages of the disclosed embodiments will be apparent from the specification and figures. The benefits and/or advantages may be individually provided by the various embodiments and features of the specification and drawings disclosure, and need not all be provided in order to obtain one or more of the same.

In one general aspect, the techniques disclosed here feature a hair growth inhibition apparatus that includes a sheet member capable of being attached to skin, a first temperature sensor that is disposed on the sheet member and detects a temperature of the skin, and a first light emitting element that is disposed on the sheet member and emits light toward the skin in accordance with a first temperature detected by the first temperature sensor.

According to the hair growth inhibition apparatus of the present disclosure, the burden on the user in the hair growth inhibition process can be reduced.

These general and specific aspects may be implemented using a system, a method, and a computer program, and any combination of systems, methods, and computer programs.

### BRIEF DESCRIPTION OF THE DRAWINGS

Fig. 1 is a diagram illustrating an example of the structure of a hair growth inhibition apparatus according to a first embodiment of the present disclosure;
Fig. 2 is a diagram illustrating an example of the appearance of a hair growth inhibition apparatus according to a second embodiment of the present disclosure and the manner in which the hair growth inhibition apparatus is used;
Fig. 3 is a diagram illustrating an example of the structure of a sheet device according to the second embodiment;
Fig. 4 is a diagram illustrating an example of the schematic structure of a temperature sensor according to the second embodiment;
Fig. 5 is a diagram illustrating an example of the functional structure of the hair growth inhibition apparatus according to the second embodiment;
Fig. 6 is a diagram illustrating an example of block information table according to the second embodiment;
Fig. 7 is a flowchart of an example of an operation of the hair growth inhibition apparatus according to the second embodiment; and
Fig. 8 is a diagram illustrating another example of the appearance of the hair growth inhibition apparatus according to the second embodiment of the present disclosure and the manner in which the hair growth inhibition apparatus is used.

### DETAILED DESCRIPTION

### Underlying Knowledge Forming Basis of the Present Disclosure

When a user uses the apparatus according to the related art, the user grips the handle to lift the housing, and places the exit opening on the area to be subjected to the hair growth inhibition process. However, such an operation is difficult to perform on some areas. Also, in the case where the area to be subjected to the hair growth inhibition process is relatively large, the user needs to repeatedly move the exit opening by small amounts over the area to be subjected to the hair growth inhibition process. Such an operation poses a large physical and temporal burden on the user. Thus, according to the related art, a large burden is placed on the user.

Embodiments of the present disclosure will now be described with reference to the drawings.

### First Embodiment

A first embodiment of the present disclosure is an example of the basic embodiment of the present disclosure.

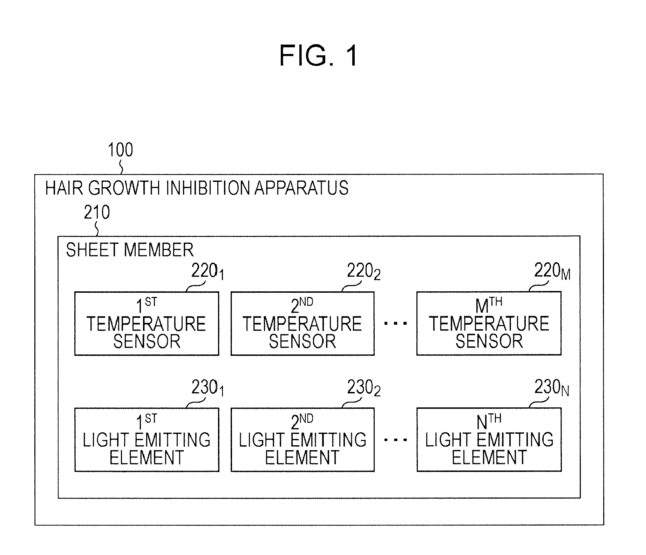

Fig. 1 is a diagram illustrating an example of the structure of a hair growth inhibition apparatus according to the present embodiment.

Referring to Fig. 1, a hair growth inhibition apparatus 100 is an apparatus that performs a hair growth inhibition process on skin by using light. The hair growth inhibition apparatus 100 includes a sheet member 210, 1^{st} to M^{th} temperature sensors 220₁ to 220_{M}, and 1^{st} to N^{th} light emitting elements 230₁ to 230_{N}.

The sheet member 210 is capable of being closely attached to skin.

Each of the 1^{st} to M^{th} temperature sensors 220₁ to 220_{M} is arranged on the sheet member 210, and detects the temperature of the skin in a region therearound.

Each of the 1^{st} to N^{th} light emitting elements 230₁ to 230_{N} is disposed on the sheet member 210 at a location corresponding to the location of the corresponding one of the 1^{st} to M^{th} temperature sensors 220₁ to 220_{M}. Each of the 1^{st} to N^{th} light emitting elements 230₁ to 230_{N} performs an operation of irradiating the skin with light for the hair growth inhibition process in a region around the corresponding temperature sensor 220 in accordance with the detection result of the corresponding temperature sensor 220.

The hair growth inhibition apparatus 100 having the above-described structure is capable of irradiating skin with light while the sheet member 210 is attached to the skin. Furthermore, the hair growth inhibition apparatus 100 is capable of irradiating each part of the skin with light in accordance with the temperature of that part. Accordingly, the hair growth inhibition apparatus 100 can perform the hair growth inhibition process while the sheet member 210 is left attached to the skin. Thus, the burden on the user in the hair growth inhibition process can be reduced.

In the hair growth inhibition apparatus 100, a single temperature sensor 220 may be disposed on the sheet member 210 instead of the plurality of temperature sensors 220. Also, in the hair growth inhibition apparatus 100, a single light emitting element 230 may be disposed on the sheet member 210 instead of the plurality of light emitting elements 230.

### Second Embodiment

A second embodiment of the present disclosure is a specific example in which the present disclosure is applied to a small sheet that can be attached to the skin on the face.

### Appearance and Structure of Hair growth inhibition Apparatus

First, the appearance and structure of a hair growth inhibition apparatus according to the present embodiment will be described.

### Appearance of Hair growth inhibition Apparatus

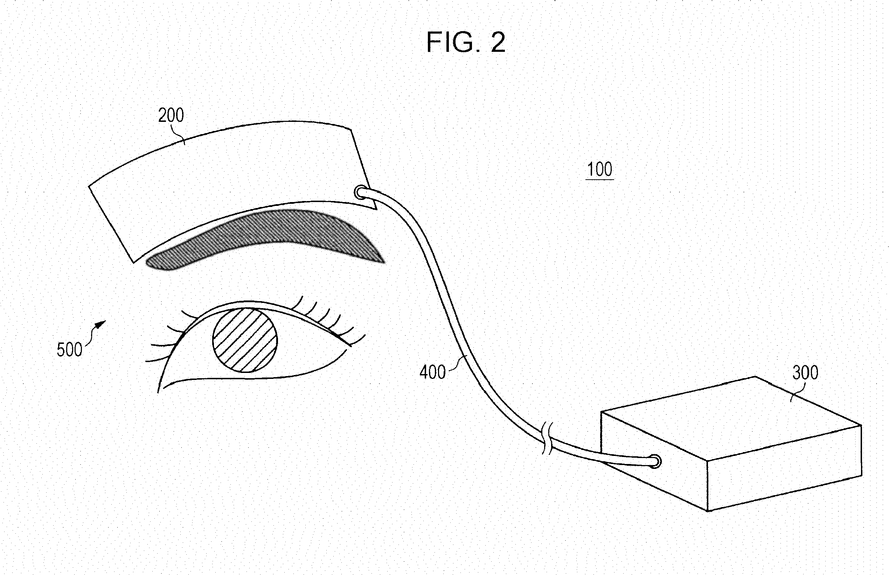

Fig. 2 is a diagram illustrating an example of the appearance of the hair growth inhibition apparatus according to the present embodiment and the manner in which the hair growth inhibition apparatus is used.

As illustrated in Fig. 2, the hair growth inhibition apparatus 100 includes a sheet device 200 and a control unit 300.

The sheet device 200 is, for example, a rectangular sheet-shaped device that is slightly longer than an eyebrow.

The sheet device 200 includes an elastic and flexible sheet member as a base material. The sheet device 200 is configured such that the state in which the sheet device 200 is closely attached to skin 500 can be maintained by surface tension. Therefore, as illustrated in Fig. 2, for example, the sheet device 200 can be attached to the skin in a region above an eyebrow while being deformed so that one long side thereof extends along the upper contour of the eyebrow. The sheet member may be made of, for example, an energy ray-cured composition containing an acryloyl group-terminated urethane polymer and an acrylic monomer (see Japanese Unexamined Patent Application Publication No. 2013-168575). To ensure sufficient adhesion, a biocompatible adhesive, such as a spirit gum, a silicone adhesive, or a latex adhesive, may be additionally used.

A plurality of temperature sensors and a plurality of light emitting elements (not shown) are arranged on a surface of the sheet member, which is the base member, at a side at which the sheet member is closely attached to the skin. The detailed structure of the sheet device 200 including the arrangement of the temperature sensors and light emitting elements will be described below.

The sheet device 200 may also be applied to another part of the face, such as the part where the beard grows.

The control unit 300 is a device protected by a housing made of a material such as plastic. As described below, the control unit 300 has a function of controlling the operation of each temperature sensor and each light emitting element. The control unit 300 is connected to the sheet device 200 by a cable 400.

Although not illustrated, the cable 400 includes signal lines that connect the control unit 300 to each of the temperature sensors and each of the light emitting elements. The length of the cable 400 may be such that, for example, the control unit 300 can be placed in a pocket of the user's clothes in the state in which the sheet device 200 is placed on the user's face.

### Structure of Sheet Device

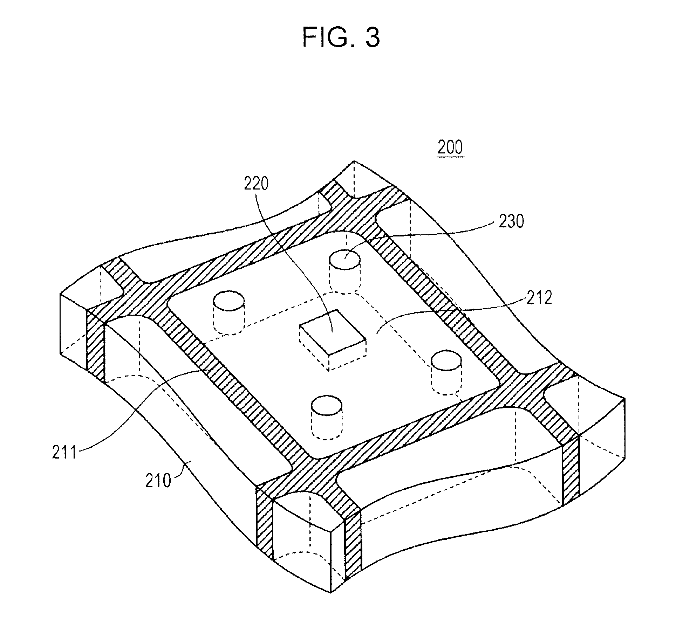

Fig. 3 is a diagram illustrating an example of the structure of the sheet device 200. Here, a portion of the entire sheet device 200 is illustrated.

Referring to Fig. 3, the sheet device 200 is structured such that a temperature sensor 220 and light emitting elements 230 are embedded in a sheet member 210.

The temperature sensor 220 has a temperature detection surface that is exposed at a surface of the sheet member 210 that is closely attachable to the skin (hereinafter referred to as an "inner surface"). Thus, in a state in which the sheet device 200 is attached to the skin, the temperature sensor 220 detects the temperature of the skin in a region around the temperature sensor 220. The temperature sensor 220 may be, for example, one in which an organic molecular layer of an organic thin-film transistor is formed of a phthalocyanine nano-size structure (see, for example, International Publication No. 2013/151128).

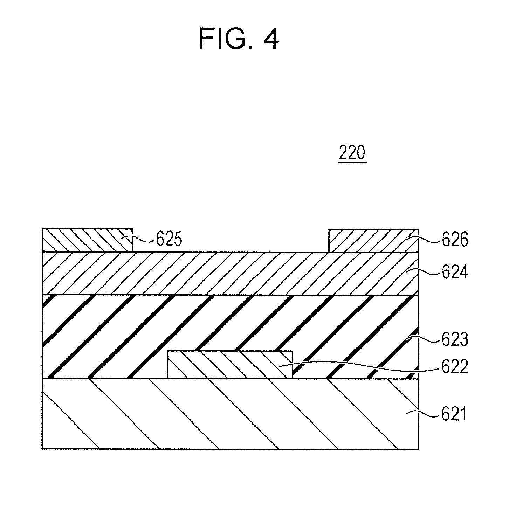

Fig. 4 is a diagram illustrating an example of the schematic structure of the temperature sensor 220 in the case in which an organic thin-film transistor is used.

Referring to Fig. 4, the temperature sensor 220 is structured such that a gate insulating layer 623 and an organic molecule layer 624 are stacked on a gate electrode layer 622 arranged on a surface of an elastic base member 621. Furthermore, the temperature sensor 220 is structured such that a source electrode 625 and a drain electrode 626 are arranged separately from each other on a surface of the organic molecule layer 624. A portion of the sheet member 210 may constitute the elastic base member 621 of the temperature sensor 220.

Each of the light emitting elements 230 illustrated in Fig. 3 has a light emitting surface that is exposed at the inner surface of the sheet member 210. Thus, in the state in which the sheet device 200 is attached to the skin, each light emitting element 230 emits predetermined light that has a hair growth inhibition effect toward the skin in a region around the light emitting element 230. Each light emitting element 230 is a light emitting diode (LED) element or a light emitting unit including a plurality of LED elements that emits predetermined light for the hair growth inhibition process. Each light emitting element 230 may be, for example, an organic LED element formed by printing by using a light emitting polymer described in Japanese Unexamined Patent Application Publication No. 2013-179107.

The predetermined light having a hair growth inhibition effect is, for example, light in a near-infrared region having a wavelength of 800 to 1000 nm and an intensity that realizes an amount of radiation of 10 mW/cm² on the surface of the skin. In this case, the distribution (density) of the light emitting elements 230 arranged on the sheet member 210 is such that the amount of radiation of 10 mW/cm² can be realized over the entire skin area of each block 212.

The above-mentioned amount of radiation is smaller than that in the related art. However, even when the amount of radiation is small, stress due to generated heat can be applied to melanin of hair roots in a dermal layer. The hair roots can be inactivated and hair growth can be inhibited by increasing the accumulated amount of radiation by, for example, repeating the radiation over several days to increase the process time (see "Skin Improvement and Hair Growth Suppression with Low-Power Xenon Flash" written by Masato Kinoshita, Masako Yamasaki, Kaori Naganuma, Kaname Okuno, Takashi Matsuzaki, and Toshitatsu Nogita in Panasonic Electric Works technical report Vol. 58, No. 2, Panasonic Electric Works, June 2010, p. 13-18)

As illustrated in Fig. 3, the sheet member 210 includes a grid-shaped light-shielding wall 211 that divides the sheet member 210 into a plurality of segments. The sheet device 200 includes a single temperature sensor 220 and four light emitting elements 230 in each of the segments (hereinafter referred to as "blocks") 212 into which the sheet device 200 is divided by the light-shielding wall 211. The size of each block 212 is, for example, 5 mm to 10 mm square.

The light-shielding wall 211 is made of a material having a low optical transparency, such as a material obtained by adding carbon, which is a biocompatible material, to the material of the sheet member 210. The height of the light-shielding wall 211 is equal to the thickness of the sheet member 210. Thus, the light-shielding wall 211 has a function of preventing light from passing therethrough between the blocks.

The light-shielding wall 211 having the above-described structure prevents light emitted from the light emitting elements 230 in each block 212 from being incident on the skin in another block 212. The light-shielding wall 211 can be formed by, for example, a method for manufacturing the sheet member 210 in multiple steps as described in Japanese Unexamined Patent Application Publication No. 2013-168575.

As described below, in the hair growth inhibition apparatus 100, the emission of light is simultaneously started in all of the blocks 212. Then, in each block 212, the emission of light is stopped when the skin temperature in the block 212 becomes higher than or equal to a predetermined threshold. The light-shielding wall 211 prevents light emitted from a block 212 in which the emission of light has not yet been stopped from leaking into a block 212 in which the emission of light has been stopped. Thus, in the hair growth inhibition apparatus 100, owing to the light-shielding wall 211, the emission of light toward the skin can be turned on and off in units of blocks with high accuracy.

In the present embodiment, it is assumed that the sheet device 200 includes L blocks 212 having approximately the same size. The operation of the sheet device 200 is controlled for each of the blocks 212 that are separated from each other by the light-shielding wall 211.

### Functional Structure of Hair Growth Inhibition Apparatus

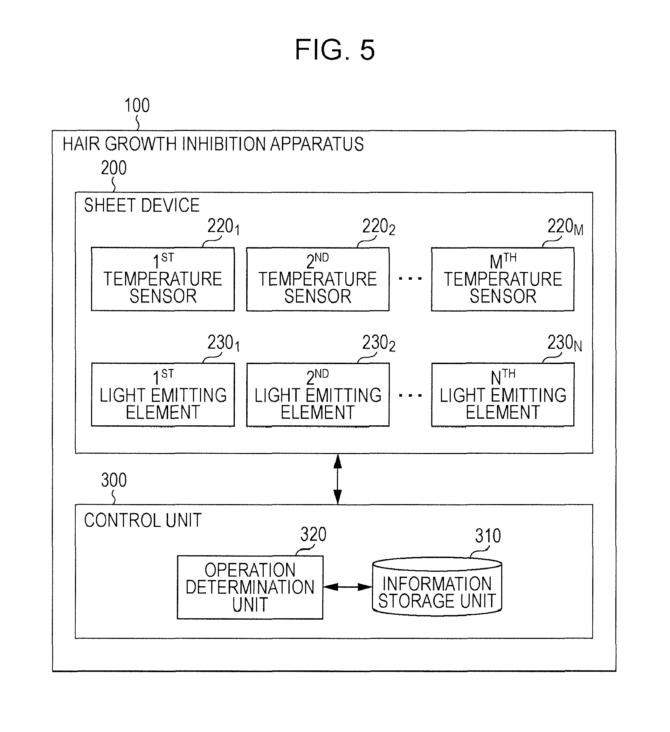

Fig. 5 is a diagram illustrating an example of the functional structure of the hair growth inhibition apparatus 100.

Referring to Fig. 5, the hair growth inhibition apparatus 100 includes 1^{st} to M^{th} temperature sensors 220₁ to 220_{M} and 1^{st} to N^{th} light emitting elements 230₁ to 230_{N} arranged on the sheet device 200, and an information storage unit 310 and an operation determination unit 320 included in the control unit 300.

The information storage unit 310 stores a block information table in advance. The block information table shows which temperature sensor 220 and which light emitting elements 230 belong to each of the above-described blocks 212.

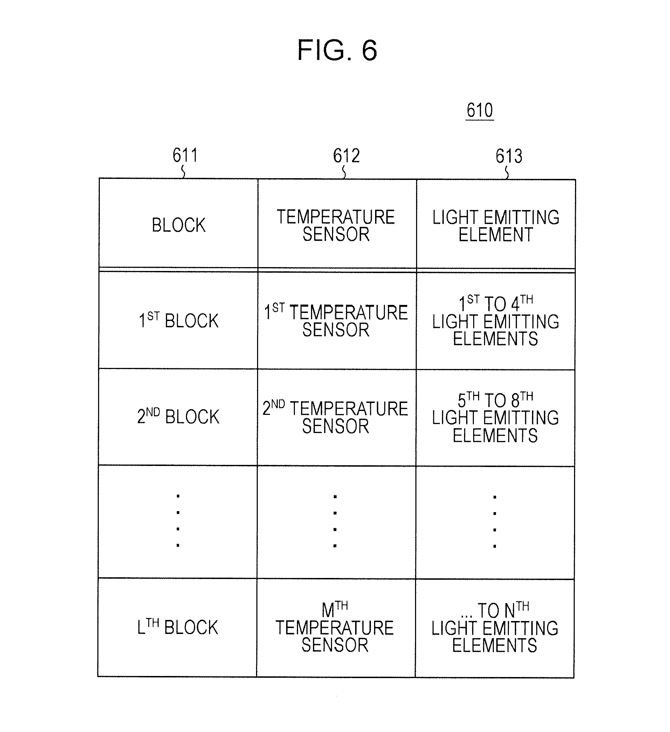

Fig. 6 shows an example of a block information table.

As illustrated in Fig. 6, a block information table 610 contains, in association with identification information 611 of each block 212, identification information 612 of a single temperature sensor 220 arranged in the block 212 and identification information 613 of four light emitting elements 230 arranged in the block 212.

For example, the skin area in which the first temperature sensor 220₁ detects the temperature corresponds to the skin area in which the first to fourth light emitting elements 230₁ to 230₄ arranged around the first temperature sensors 220₁ emit light. Therefore, when the operation the light emitting elements 230 is controlled on the basis of the detection results obtained by the temperature sensors 220 in units of blocks 212, light can be emitted in accordance with the temperature of each part of the skin.

The operation determination unit 320 illustrated in Fig. 5 is connected to each of the 1^{st} to M^{th} temperature sensors 220₁ to 220_{M} and each of the 1^{st} to N^{th} light emitting elements 230₁ to 230_{N} by the cable 400 (see Fig. 2) that connects the control unit 300 and the sheet device 200 to each other and signal lines (not shown) embedded in the sheet device 200.

Accordingly, the operation determination unit 320 is capable of outputting a control signal to each temperature sensor 220 to control the operation of the temperature sensor 220, and receiving a detection value output from each temperature sensor 220. The operation determination unit 320 is also capable of outputting a control signal to each light emitting element 230 to control the operation of the light emitting element 230.

In the present embodiment, the operation determination unit 320 controls the light emitting elements 230 in each block in the same light emission pattern. The light emission pattern is, for example, that of intermittent pulse light emitted at a predetermined output level, a predetermined period, and a predetermined duty ratio. More specifically, the light emission pattern is a pattern in which, for example, an output period in which light is emitted from each light emitting element 230 at a maximum power for 3 seconds and an output stop period in which no light is emitted from each light emitting element 230 for 5 seconds are repeated. In the output period, a light emission period of 0.05 seconds and a light non-emission period of 0.05 seconds may be repeated.

The operation determination unit 320 determines the operation of the light emitting elements 230 arranged in each block 212 on the basis of the detection value obtained by the temperature sensor 220 arranged in that block 212. More specifically, when the skin temperature becomes higher than or equal to a predetermined threshold, the operation determination unit 320 stops the emission of light from the corresponding light emitting elements 230. The predetermined threshold is preset in the operation determination unit 320 or the information storage unit 310. The predetermined threshold is, for example, 42 degrees.

Although not illustrated, the control unit 300 includes a central processing unit (CPU), a read only memory (ROM) that stores a control program, and a working memory, such as a random access memory (RAM). In this case, the function of each part of the control unit 300 is realized by causing the CPU to execute the control program.

In addition, although not illustrated, the control unit 300 includes a power supply unit and an operation unit including a key switch or the like. The power supply unit supplies electric power for operating the CPU and the sheet device 200. The operation unit receives commands for various operations including an operation of starting the hair growth inhibition process from the user.

With the above-described structure, the hair growth inhibition apparatus 100 is capable of irradiating skin with light while the sheet member 210 is attached to the skin. Also, the hair growth inhibition apparatus 100 is capable of irradiating each part of the skin with light in accordance with the temperature of that part to prevent cold burn or the like.

### Operation of Hair Growth Inhibition Apparatus

Next, the operation of the hair growth inhibition apparatus 100 will be described.

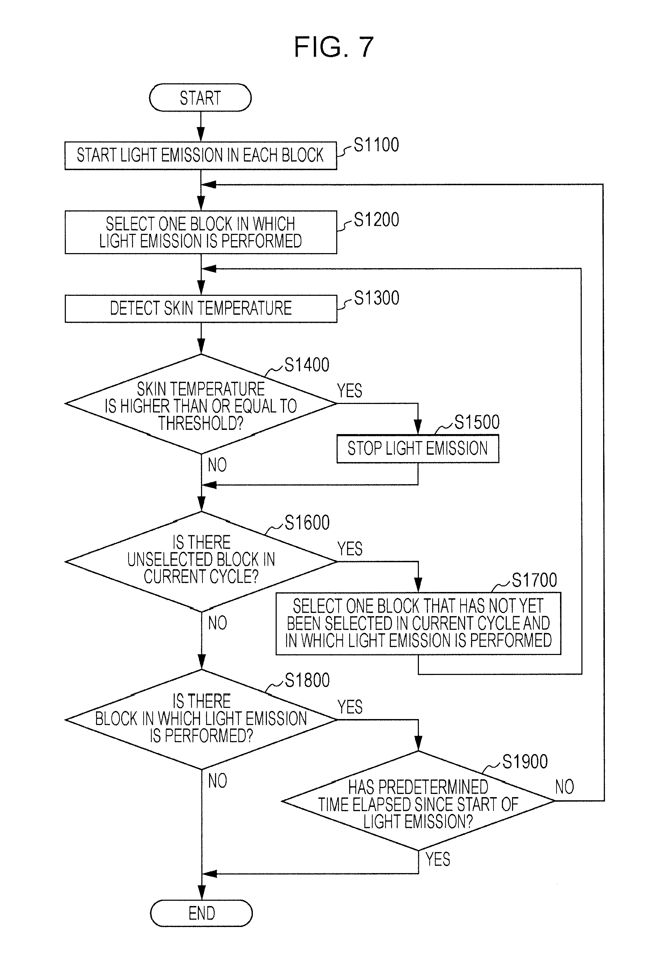

Fig. 7 is a flowchart of an example of the operation of the hair growth inhibition apparatus 100.

When the user performs an operation of issuing an operation start command while the sheet device 200 is attached to the user's skin, the hair growth inhibition apparatus 100 starts the following process.

First, in Step S1100, the operation determination unit 320 starts the light emission in each block 212 over the entire region of the sheet device 200.

Then, in Step S1200, the operation determination unit 320 selects one of the blocks 212 in which the light emission is being performed. The operation determination unit 320 regards an interval between the points at which Step 1800 is performed as a single process cycle.

Then, in Step S1300, the temperature sensor 220 in the block 212 that is being selected detects the skin temperature.

Then, in Step S1400, the operation determination unit 320 determines whether or not the detected skin temperature is higher than or equal to the predetermined threshold (42 degrees). When the skin temperature is higher than or equal to the predetermined threshold (YES in S1400), the operation determination unit 320 proceeds the process to Step S1500. When the skin temperature is lower than the predetermined threshold (NO in S1400), the operation determination unit 320 proceeds the process to Step S1600.

In Step S1500, the operation determination unit 320 stops the light emission in the selected block, and proceeds the process to Step S1600.

In Step S1600, the operation determination unit 320 determines whether or not there is a block 212 that has not yet been selected in the current cycle and in which the light emission is being performed. If there is an unselected block 212 in which the light emission is being performed (YES in S1600), the operation determination unit 320 proceeds the process to Step S1700. In the case where there is no unselected block 212 in which the light emission is being performed (NO in S1600), the operation determination unit 320 proceeds the process to Step S1800.

In Step S1700, the operation determination unit 320 selects one of the unselected blocks 212 in which the light emission is being performed, and returns the process to Step S1300.

In Step S1800, the operation determination unit 320 determines whether or not there is a block 212 in which the light emission is being performed. When there is a block 212 in which the light emission is being performed (YES in S1800), the operation determination unit 320 proceeds the process to Step S1900. When there is no block 212 in which the light emission is being performed (NO in S1800), the operation determination unit 320 ends the process.

In Step S1900, the operation determination unit 320 determines whether or not a predetermined time has elapsed since the light emission over the entire region of the sheet device 200 was started in Step S1100. The predetermined time is a time set in the operation determination unit 320 or the information storage unit 310 in advance as a time short enough to prevent cold burn. Time measurement is performed by using, for example, a timer (not shown) mounted in the control unit 300.

When it is determined that the predetermined time has not yet elapsed (NO in S1900), the operation determination unit 320 returns the process to Step S1200. When it is determined that the predetermined time has elapsed (YES in S1900), the operation determination unit 320 ends the process.

Thus, the hair growth inhibition apparatus 100 performs the light emission in each block while monitoring the skin temperature. With this operation, the hair growth inhibition apparatus 100 is capable of performing the light emission as actively as possible while preventing the skin from being damaged due to a temperature increase or the like.

The user uses, for example, the sheet device 200 once every day for several days to several weeks. As a result, the hair roots are inactivated and hair growth is inhibited.

### Effects of Hair Growth Inhibition Apparatus

As described above, with the hair growth inhibition apparatus 100 according to the present embodiment, the skin can be irradiated with light while the sheet member 210 is attached to the skin. In addition, the light emission can be stopped at the time when the skin temperature reaches the predetermined threshold. Accordingly, the hair growth inhibition apparatus 100 is capable of performing the hair growth inhibition process safely while the sheet member 210 is left attached to the skin. Therefore, the burden on the user in the hair growth inhibition process can be reduced.

In addition, with the hair growth inhibition apparatus 100 according to the present embodiment, the hair growth inhibition process can be performed without the use of hands. Therefore, the user can perform other activities or operations while using the hair growth inhibition apparatus 100.

In addition, the hair growth inhibition apparatus 100 according to the present embodiment can be used continuously for a long time. Therefore, the hair growth inhibition effect can be achieved even when the amount of radiation is small. In other words, with the hair growth inhibition apparatus 100 according to the present embodiment, not only can the stress on the skin be reliably reduced, but the maximum current value of the entire apparatus required to emit light can be reduced.

The arrangement of the blocks 212, the blocks 212 to be controlled, the arrangement and number of the temperature sensors 220 and the light emitting elements 230 in each block, and the shape of the blocks 212 are not limited to those in the above-described example.

### Other Examples of Light Emission Pattern

The stress on the skin can be reduced by intermittently emitting light as in the above-described light emission pattern. In this case, each light emitting element 230 has periods in which light is emitted and periods in which light is not emitted.

Accordingly, the operation determination unit 320 may determine the operation of each light emitting element 230 so that electricity supplying periods (light emitting periods) in which a current is supplied to the light emitting elements 230 do not overlap in at least some of the 1^{st} to N^{th} light emitting elements 230₁ to 230_{N}.

For example, the operation determination unit 320 may divide the blocks 212 into two groups such that the blocks 212 belonging to the respective groups are arranged in a checkerboard pattern, and control the operation timing of the light emitting elements 230 so that the light output periods of the blocks in the two groups do not overlap. Alternatively, in the case where a plurality of light emitting elements 230 are arranged in each block, the operation determination unit 320 shifts the light emitting times of the light emitting elements 230 with respect to each other in each block.

Thus, with the hair growth inhibition apparatus 100, the required maximum current value can be further reduced.

### Control of Amount of Radiation Based on Skin Condition

An appropriate amount of radiation differs in accordance with the skin condition, such as the skin color or the amount of moisture in the skin. For example, since dark skin more easily absorbs optical energy than light skin, the hair growth inhibition process for dark skin may be performed with a relatively small amount of radiation. In addition, the amount of radiation may be reduced in areas where moles, blemishes, etc., are present and the skin is darker than in other areas.

Accordingly, the hair growth inhibition apparatus 100 may include, for example, skin condition sensors that are arranged on the sheet member 210 to detect information related to the skin condition, and change the amount of radiation of light for each light emitting element 230 in accordance with the detection results of the skin condition sensors.

In the case where the skin condition sensors are color tone sensors, the operation determination unit 320 sets the outputs of the light emitting elements 230 to higher levels for darker skin colors. The amount of radiation can be controlled for the entire region of the sheet device 200 together or for each segment (block 212). In the latter case, the hair growth inhibition apparatus 100 needs to include a color tone sensor for each segment and determine the amount of radiation for each segment on the basis of the detection result of the color tone sensor. To determine the amount of radiation, the operation determination unit 320 may refer to a table in which color tones and amounts of radiation (drive current values) to be set for the light emitting elements 230 are stored in association with each other.

Each color tone sensor may be, for example, a device described in "Trend in Research on Organic Imaging Devices" written by Satoshi Aihara and Misao Kubota in NHK STRL R&D No. 132, NHK Science & Technology Research Laboratories, March 2012, pp. 4-11. In this device, three primary colors, R, G, and B, are captured with organic semiconductors. The light receiving surface of each color tone sensor may be at a position closer to the central region of the sheet member 210 than the inner surface of the sheet member 210. For example, in the state in which the inner surface of the sheet member 210 is closely attached to the skin, the light receiving surface may be at about 100 micrometers away from the skin.

The light emitting elements 230, for example, may be used as light sources that are necessary to detect the skin color with the color tone sensors. Alternatively, additional light emitting elements that emit white light and that are also arranged on the sheet member 210 may be used.

### Another Example of Sheet Shape

In the above-described embodiment, the sheet device 200 is a small sheet that can be attached to the skin on the face. However, the size and shape of the sheet device 200 is not limited to this.

For example, the sheet device 200 may have a three-dimensional shape that follows the shape of the face or body.

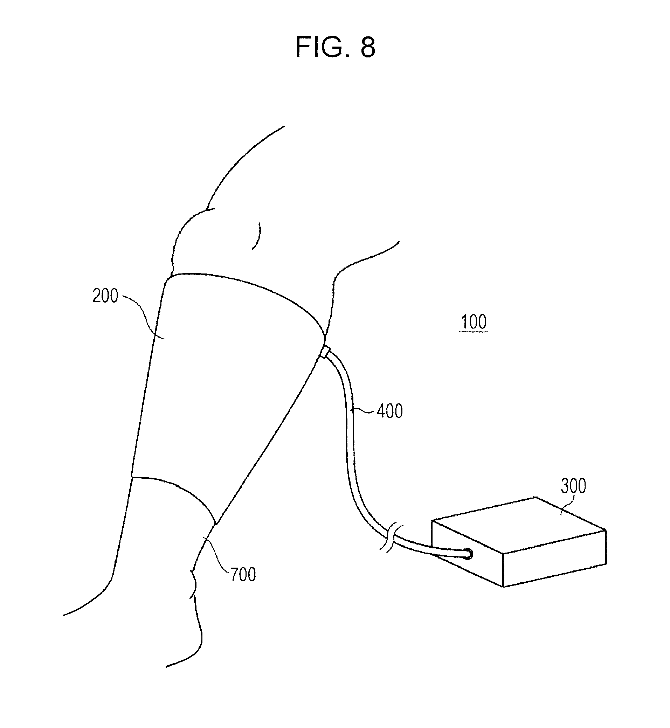

Fig. 8 is a diagram illustrating another example of the appearance of the hair growth inhibition apparatus 100 and the manner in which the hair growth inhibition apparatus 100 is used. Fig. 8 corresponds to Fig. 2.

As illustrated in Fig. 8, the sheet device 200 has, for example, a cylindrical shape so as to cover a leg (shin and calf) 700. Since the sheet member 210 is elastic and flexible, the sheet device 200 deforms along the shape of the leg 700 and is closely attached to the entire region of the leg 700. The state in which the sheet device 200 is closely attached to the leg 700 is maintained by the elasticity of the sheet member 210, frictional force between the sheet member 210 and the skin, and surface tension.

With this hair growth inhibition apparatus 100, a large skin area can be simultaneously subjected to the hair growth inhibition process.

Even when the sheet device 200 has a planar shape, since the sheet device 200 is elastic and flexible, it can be closely attached to the face or body having a three-dimensional shape to a certain degree. For example, a sheet device 200 with a size of about 20 cm × 10 cm is suitable for the hair growth inhibition process on the back of the neck, a forearm, or a lower abdominal region.

There may be a case in which the sheet device 200 includes a region in which the hair growth inhibition process is not performed. For example, when the sheet device 200 is configured to cover the entire forehead including eyebrows, the eyebrow regions may be excluded from an area subjected to the hair growth inhibition process. The eyebrow regions may either be set in advance, or be input to the control unit 300 by the user through a personal computer (PC), a smart phone, or the like. Alternatively, shapes of eyebrows may be accumulated in, for example, a server via the Internet or the like, and the user may select and download one of the shapes of the eyebrows into the control unit 300. In this case, the control unit 300 is, of course, required to have a communicating function.

### Other Modifications

The amount of light emitted from the sheet device 200 toward the skin may be larger than that in the above-described example depending on the output performance of the light emitting elements 230 and the heat resistance of the sheet device 200. In this case, similar to the related art, the hair growth inhibition apparatus 100 is capable of destroying the hair root cells, and the hair growth inhibition (or hear removing) process can be more effectively performed.

At least one of the functions of the control unit 300 according to the above-described second embodiment may be provided by a device having another function as the main function, such as a mobile phone.

Alternatively, the above-described functions may be provided in a network server. In other words, at least one of the functions of the hair growth inhibition apparatus may be realized through cloud computing. In this case, the operation determination unit needs to include at least a communication unit. More specifically, the operation determination unit needs to transmit data representing the skin temperature to the server and receive the timing for stopping the light emission.

In addition, the hair growth inhibition apparatus 100 may include operation determination units 320 for the respective blocks, each operation determination unit 320 operating the corresponding block. In this case, for example, each operation determination unit 320 may be a comparator circuit that compares the output signal of the corresponding temperature sensor 220 with a predetermined threshold and outputs the result of the comparison to the corresponding light emitting elements 230 as a control signal.

A hair growth inhibition apparatus according to the present disclosure includes a sheet member capable of being attached to skin, a first temperature sensor that is disposed on the sheet member and detects a temperature of the skin, and a first light emitting element that is disposed on the sheet member and emits light toward the skin in accordance with a first temperature detected by the first temperature sensor.

In the above-described hair growth inhibition apparatus, the first light emitting element may stop emitting the light when the first temperature has become higher than or equal to a predetermined threshold.

In the above-described hair growth inhibition apparatus, the sheet member may be divided into a plurality of segments, each of the plurality of segments having a temperature sensor which detects a temperature of the skin and a light emitting element which emits light toward the skin in accordance with the temperature detected by the temperature sensor, the plurality of segments may include a first segment having the first temperature sensor and the first light emitting element, and the hair growth inhibition apparatus may further include an operation determination unit that determines an operation of the light emitting element on the basis of the detected temperature.

In the above-described hair growth inhibition apparatus, the sheet member may include a light-shielding wall that divides the sheet member into the plurality of segments and prevents the light emitted from each segment from being incident on the skin in another segment.

In the above-described hair growth inhibition apparatus, the operation determination unit may determine the operation of the light emitting element so that an electricity supply period in which a current is supplied to the light emitting element do not overlap with an electricity supply period in which a current is supplied to another light emitting element.

In the above-described hair growth inhibition apparatus, the first light emitting element intermittently may emit the light at a predetermined output level, for a predetermined period, and at a predetermined duty ratio.

The above-described hair growth inhibition apparatus may further include a skin condition sensor that is disposed on the sheet member and detects information related to a condition of the skin, and the first light emitting element may vary an amount of the emitted light in accordance with a result detected by the skin condition sensor.

A hair growth inhibition method according to the present disclosure includes detecting, with a temperature sensor disposed on a sheet member capable of being attached to skin, a temperature of the skin, and operating a light emitting element disposed on the sheet member in accordance with the detected temperature and emitting the light toward the skin.

The present disclosure is effective as a hair growth inhibition apparatus and a hair growth inhibition method capable of reducing the burden on the user in a hair growth inhibition process.

## Claims

1. A hair growth inhibition apparatus comprising:
a sheet member capable of being attached to skin;
a first temperature sensor that is disposed on the sheet member and detects a temperature of the skin; and
a first light emitting element that is disposed on the sheet member and emits light toward the skin in accordance with a first temperature detected by the first temperature sensor.

2. The hair growth inhibition apparatus according to Claim 1, wherein the first light emitting element stops emitting the light when the first temperature has become higher than or equal to a predetermined threshold.

3. The hair growth inhibition apparatus according to Claim 1,
wherein the sheet member is divided into a plurality of segments, each of the plurality of segments having a temperature sensor which detects a temperature of the skin and a light emitting element which emits light toward the skin in accordance with the temperature detected by the temperature sensor,
wherein the plurality of segments including a first segment having the first temperature sensor and the first light emitting element, and
wherein the hair growth inhibition apparatus further comprises an operation determination unit that determines an operation of the light emitting element on the basis of the detected temperature.

4. The hair growth inhibition apparatus according to Claim 3, wherein the sheet member includes a light-shielding wall that divides the sheet member into the plurality of segments and prevents the light emitted from each segment from being incident on the skin in another segment.

5. The hair growth inhibition apparatus according to Claim 3, wherein the operation determination unit determines the operation of the light emitting element so that an electricity supply period in which a current is supplied to the light emitting element do not overlap with an electricity supply period in which a current is supplied to another light emitting element.

6. The hair growth inhibition apparatus according to Claim 1, wherein the first light emitting element intermittently emits the light at a predetermined output level, for a predetermined period, and at a predetermined duty ratio.

7. The hair growth inhibition apparatus according to Claim 1, further comprising:
a skin condition sensor that is disposed on the sheet member and detects information related to a condition of the skin,
wherein the first light emitting element varies an amount of the emitted light in accordance with a result detected by the skin condition sensor.

8. A hair growth inhibition method comprising:
detecting, with a temperature sensor disposed on a sheet member capable of being attached to skin, a temperature of the skin; and
operating a light emitting element disposed on the sheet member in accordance with the detected temperature and emitting the light toward the skin.
